# EUROPEAN PATENT APPLICATION

(11) **EP 4 144 828 A1**
(43) Date of publication of application: **08.03.2023**
(21) Application number: 21796545.8
(22) Date of filing: 12.04.2021
(51) Int. Cl.: C12M 3/00

(54) **INCUBATOR**

(30) Priority: 30.04.2020 JP 2020080007
(71) Applicant: Airex Co., Ltd., Nagoya-shi Aichi 453-0015 (JP)
(72) Inventor: KAWASAKI, Koji, Nagoya-shi Aichi 453-0015 (JP); OGATA, Yoshitaka, Nagoya-shi Aichi 453-0015 (JP); NISHIWAKI, Hideo, Nagoya-shi Aichi 453-0015 (JP); KAKUDA, Daisuke, Nagoya-shi Aichi 453-0015 (JP); MASUDOME, Jun, Nagoya-shi Aichi 453-0015 (JP); YAZAKI, Yukihiro, Nagoya-shi Aichi 453-0015 (JP); GUO, Zhiqiang, Nagoya-shi Aichi 453-0015 (JP); KITANO, Tsukasa, Nagoya-shi Aichi 453-0015 (JP)
(74) Representative: Lederer & Keller Patentanwälte Partnerschaft mbB
(86) International application number: PCT/JP2021/015153
(87) International publication number: WO 2021/220784

(57) **Abstract**

The present invention can provide an incubator having not only a favorable cell culture function but also an autonomous decontamination function that saves the need for connection to an external decontamination device.

The incubator is provided with a culture vessel, a circulating means, a temperature-regulating means, and a gas supplying means for supplying a decontamination gas or a humidifying water vapor. The gas supplying means includes a compressed gas generating means, a decontamination solution supplying means, a water supplying means, a mixed gas/liquid regulator, and a vaporizer for generating a decontamination gas or a water vapor. The gas supplying means acts as a decontamination means for supplying the decontamination gas to the air circulating in the circulation passage during an autonomous decontamination before cells are cultured, and the gas supplying means acts as a humidifying means for humidifying the air circulating in the circulation passage when cells are cultured after the autonomous decontamination.

## Description

### TECHNICAL FIELD

The present invention relates to an incubator and, more particularly, to an incubator including a vaporizer for generating a decontamination gas or a water vapor and having not only a cell culture function but also an autonomous decontamination function.

### BACKGROUND ART

The recent advanced technology in the field of regenerative medicine has led to wide use of incubators for cell culture. Cells are cultured inevitably in a culture environment suitable for each type of cell by setting the temperature and humidity conditions, or as needed, regulating the carbon dioxide gas concentration, the nitrogen gas concentration and other parameters inside an incubator.

A process of setting the temperature condition inside an incubator involves regulating the indoor temperature by radiation heat from a wall surface by normally providing a hot water heater or an electric heater on a door and a wall portion such as a shelf board inside the incubator. Another process of setting the humidity condition inside the incubator involves regulating the indoor humidity by natural evaporation of reservoir water on a humidification pan normally provided inside the incubator to reserve water.

Meanwhile, a process of regulating the carbon dioxide gas concentration or the nitrogen gas concentration inside the incubator involves normally providing a carbon dioxide gas concentration sensor, a nitrogen gas concentration sensor and supply passages from a carbon dioxide gas cylinder and a nitrogen gas cylinder. In addition to these processes, such parameters can be equalized aided by air agitation from an indoor fan.

However, when the indoor temperature and the indoor humidity are regulated by radiation heat from a wall surface or air agitation, these parameters tend to be non-uniform. In particular, partial condensation can undesirably occur when the temperature and humidity values are non-uniform due to a high humidity inside an incubator.

Meanwhile, some conventional incubators fail to meet Grade A standards (Guidance on the Manufacture of Sterile Pharmaceutical Products by the Ministry of Health, Labour and Welfare of Japan) in accordance with GMP (Good Manufacturing Practice). Even a Grade A-based sterilization for the inside of an incubator unsuccessfully keeps a higher air pressure than in the external environment to maintain the sterile state. Furthermore, evaporation of reservoir water inside the incubator requires water supply from the outside, thereby allowing the supplied water to unfortunately adversely affect the sterile environment.

Thus, the following patent document 1 proposes an incubator characterized by the improvement in the precision of humidity regulation by generally regulating the temperature, with an indoor heater, a door heater, and a stage heater turned ON/OFF, and by fine-tuning the area of the water surface exposed in a humidification pan when rises in the internal humidity are likely to cause condensation. Likewise, the following patent document 2 proposes an incubator including a filter in a water supply passage from the outside of the incubator to a humidification pan.

In fact, despite suppressed condensation occurrence, the incubator in the following patent document 1 is problematic in that the indoor temperature and humidity can be non-uniform. The incubator in the following patent document 2 has a similar problem of non-uniform indoor temperature and humidity although the water reserved on the humidification pan can assuredly be sterilized. Inventors of the present invention have proposed another inventive incubator in the following patent document 3 to cope with the above-mentioned problems.

### CITATION LIST

### PATENT LITERATURE

Patent Document 1: JP-A-2008-005759
Patent Document 2: JP-A-2011-160672
Patent Document 3: WO2018-212029

### SUMMARY OF INVENTION

### TECHNICAL PROBLEM

Accordingly, the incubator provided in the above patent document 3 can be an incubator having a favorable cell culture function because it can equalize the temperature and humidity inside a culture vessel and generate no excessive condensation inside the culture vessel.

Nevertheless, in order to achieve increasingly required development in the field of regenerative medicine, requirements for incubators should include a function adapted for mass culture cycles utilizing multiple incubators. One such requirement is to reduce the complexity of a decontamination process that is essentially connected to an external decontamination device for each cell culture.

Thus, the present invention was made in view of the situation to solve the problems, and has an object to provide an incubator having not only a favorable cell culture function provided in the above patent document 3 but also an autonomous decontamination function that saves the need for connection to an external decontamination device.

### SOLUTION TO PROBLEM

To solve the aforementioned problem, inventors of the present invention have carried out an extended investigation to find that a proper amount of decontamination gas can be supplied using a circulation passage of air, by using a vaporizer capable of supplying a trace of sterilized water vapor to a circulation passage of the air inside a culture vessel as needed as a decontamination gas supplying device. Based on that technique, the present invention was accomplished.

Specifically, an incubator according to the present invention is, according to description in claim 1,
an incubator having not only a cell culture function but also an autonomous decontamination function, the incubator including:
a culture vessel (20) having an insulating door (20a) and an insulating wall (20b, 20c, 20d) ; a circulating means (30) having a circulation passage (32) for circulating the air inside the culture vessel;
a temperature-regulating means (40) for regulating the temperature of the air inside the culture vessel; and a gas supplying means (50) for supplying a decontamination gas or a humidifying water vapor to the air inside the culture vessel, characterized in that
the gas supplying means includes: a compressed gas generating means (51) for generating a compressed gas; a decontamination solution supplying means (56) for supplying a decontamination liquid; a water supplying means (52) for supplying water; a mixed gas/liquid regulator (53) for regulating a mixed gas/liquid composed of the compressed gas and the decontamination liquid or the water in mixture; and a vaporizer (54, 55) for vaporizing the mixed gas/liquid to generate a decontamination gas or a water vapor, characterized in that
the gas supplying means acts as a decontamination means for supplying the decontamination gas to the air circulating in the circulation passage during an autonomous decontamination before cells are cultured, and
the gas supplying means acts as a humidifying means for humidifying the air circulating in the circulation passage when cells are cultured after the autonomous decontamination.

Moreover, the present invention is, according to description in claim 2, the incubator according to claim 1, including
a decontamination gas decomposing means (80) for decomposing a decontamination gas, characterized in that
the decontamination gas decomposing means includes a branch passage (81a, 81b) branching off from the circulation passage and a catalyst means (82) for decomposing the decontamination gas in the branch passage, characterized in that
the branch passage is closed to prevent the air circulating in the circulation passage from flowing into the branch passage during an autonomous decontamination before cells are cultured, and
the branch passage is opened to allow the air circulating in the circulation passage to flow into the branch passage and allow the catalyst means to decompose the residual decontamination gas in the air when the autonomous decontamination is completed.

Furthermore, the present invention is, according to description in claim 3, the incubator according to claim 1 or 2, characterized in that
the compressed gas is a single gas or a mixed gas composed of one or more of air, carbon dioxide and nitrogen.

Moreover, the present invention is, according to description in claim 4, the incubator according to any one of claims 1 to 3, characterized in that
the amount of a decontamination gas supplied by the vaporizer is in the range of 0.1 g/min to 10 g/min, and the amount of a water vapor supplied by the vaporizer is in the range of 1 g/h to 60 g/h.

Furthermore, the present invention is, according to description in claim 5, the incubator according to any one of claims 1 to 4, characterized in that
the vaporizer (54) includes a cylindrical outer cylinder pipe (61) and a heat generator (62) provided inside the outer cylinder pipe (61) and parallel to the longitudinal direction of the outer cylinder pipe, characterized in that
the mixed gas/liquid is heated while it passes between the outer cylinder pipe and the heat generator to supply a decontamination gas or a sterilized water vapor.

Moreover, the present invention is, according to description in claim 6, the incubator according to claim 5, characterized in that
the heat generator is a heater (63) coated with a quartz glass (64).

Furthermore, the present invention is, according to description in claim 7, the incubator according to any one of claims 1 to 4, characterized in that
the vaporizer (55) includes a cylindrical outer cylinder pipe (71) and a heat generator (72) provided inside the outer cylinder pipe (71) and parallel to the longitudinal direction of the outer cylinder pipe, characterized in that
the heat generator includes stick-like heater (73) arranged in the longitudinal direction thereof and an evaporation pipe (74) helically wound along the periphery of the heater in the longitudinal direction, and
the mixed gas/liquid is heated while it passes inside the evaporation pipe to supply a decontamination gas or a sterilized water vapor.

### ADVANTAGEOUS EFFECTS OF INVENTION

According to the above configuration, the incubator of the present invention includes a culture vessel, a circulating means, a temperature-regulating means, and a gas supplying means. The culture vessel includes an insulating door and an insulating wall. The circulating means includes a circulation passage to circulate the air inside the culture vessel. The temperature-regulating means regulates the temperature of the air inside the culture vessel. The gas supplying means supplies a decontamination gas or a humidifying water vapor to the air inside the culture vessel.

In addition, the gas supplying means includes a compressed gas generating means, a decontamination solution supplying means, a water supplying means, a mixed gas/liquid regulator, and a vaporizer. The mixed gas/liquid regulator mixes a compressed gas generated by the compressed gas generating means, a decontamination liquid supplied by the decontamination solution supplying means or water supplied by the water supplying means to generate a mixed gas/liquid. The vaporizer vaporizes the mixed gas/liquid generated to generate a decontamination gas or a water vapor.

In such a configuration, the gas supplying means acts as a decontamination means for supplying the decontamination gas to the air circulating in the circulation passage during an autonomous decontamination before cells are cultured. Meanwhile, the gas supplying means acts as a humidifying means for humidifying the air circulating in the circulation passage when cells are cultured after the autonomous decontamination. Accordingly, the present invention can provide an incubator having not only a favorable cell culture function but also an autonomous decontamination function that saves the need for connection to an external decontamination device.

According to the above configuration, the incubator of the present invention includes not only the above components but also a decontamination gas decomposing means. The decontamination gas decomposing means includes a branch passage branching off from the circulation passage and a catalyst means, and the catalyst means is in the branch passage to decompose a decontamination gas.

In such a configuration, the branch passage is closed to prevent the air circulating in the circulation passage from flowing into the branch passage during an autonomous decontamination before cells are cultured. Meanwhile, the branch passage is opened to allow the air circulating in the circulation passage to flow into the branch passage and allow the catalyst means to decompose the residual decontamination gas in the air when the autonomous decontamination is completed. Accordingly, the above operational advantage can more efficiently be provided.

According to the above configuration, the compressed gas generated by the compressed gas generating means is a single gas or a mixed gas composed of one or more of air, carbon dioxide and nitrogen. Accordingly, the concentration of air, carbon dioxide or nitrogen required for the culture environment can be maintained uniformly in a prescribed range when the vaporizer generates a water vapor.

According to the above configuration, the amount of the decontamination gas supplied by the vaporizer is in the range of 0.1 g/min to 10 g/min. Accordingly, the supply of a proper amount of decontamination gas to an incubator having a small volume can provide a sufficient decontamination effect, resulting in no excessive condensation of a decontamination liquid inside a culture vessel. Accordingly, the above operational advantage can more efficiently be provided.

According to the above configuration, the amount of the water vapor supplied by the vaporizer is in the range of 1 g/h to 60 g/h when cells are cultured after the autonomous decontamination. Accordingly, the resulting stable or as-needed supply of a trace of water vapor can uniformly maintain the temperature and humidity inside the culture vessel, resulting in no excessive condensation inside the culture vessel. Accordingly, the above operational advantage can more efficiently be provided.

According to the above configuration, the vaporizer includes a cylindrical outer cylinder pipe and a heat generator. The heat generator is provided inside the outer cylinder pipe and parallel to the longitudinal direction of the outer cylinder pipe. Accordingly, the mixed gas/liquid generated by the mixed gas/liquid regulator is heated while it passes between the outer cylinder pipe and the heat generator and supplied to the inside of the culture vessel as a decontamination gas or a sterilized water vapor. Accordingly, the above operational advantage can more specifically be provided. In addition, since the water vapor generated by the vaporizer is sterilized at high temperature when cells are cultured, the vapor doesn't pass through an air filter and can directly be supplied to the inside of the culture vessel.

According to the above configuration, the heat generator included in the vaporizer may be a heater coated with quartz glass. Accordingly, there is no contamination on the water vapor generated by the vaporizer, and not only a sterile environment inside the culture vessel but also a dust-free environment can be maintained with advanced level particularly when cells are cultured.

According to the above configuration, the heat generator included in the vaporizer may include a stick-like heater arranged in the longitudinal direction thereof and an evaporation pipe helically wound along the periphery of the heater in the longitudinal direction. Accordingly, the mixed gas/liquid generated by the mixed gas/liquid regulator is heated while it passes inside the evaporation pipe and supplied to the inside of the culture vessel as a decontamination gas or a sterilized water vapor. Accordingly, the above operational advantage can more specifically be provided. In addition, since the water vapor generated by the vaporizer is sterilized at high temperature when cells are cultured, the vapor doesn't pass through an air filter and can directly be supplied to the inside of the culture vessel.

The reference numerals in parentheses of each of the above means correspond to the specific means described in the following embodiment.

### BRIEF DESCRIPTION OF DRAWINGS

FIG. 1 is a cross-sectional view illustrating the inside of an incubator according to one embodiment of the present invention seen from the side;
FIG. 2 is a cross-sectional view illustrating the inside of the incubator in FIG. 1 seen from above;
FIG. 3 is a cross-sectional view illustrating a gas supplying device included in the incubator in FIG. 1;
FIG. 4 is a cross-sectional view illustrating a vaporizer included in the gas supplying device in FIG. 3 according to one embodiment of the present invention; and
FIG. 5 is a cross-sectional view illustrating the vaporizer included in the gas supplying device in FIG. 3 according to another embodiment of the present invention.

### DESCRIPTION OF EMBODIMENTS

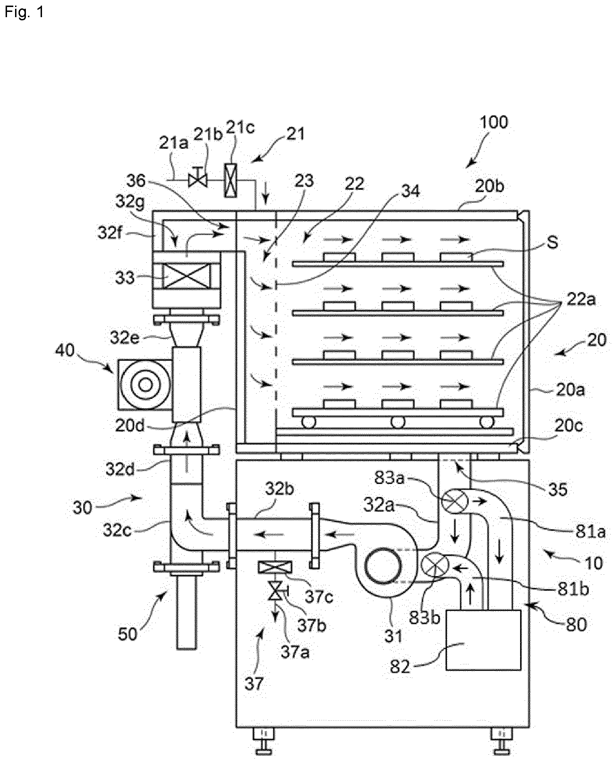

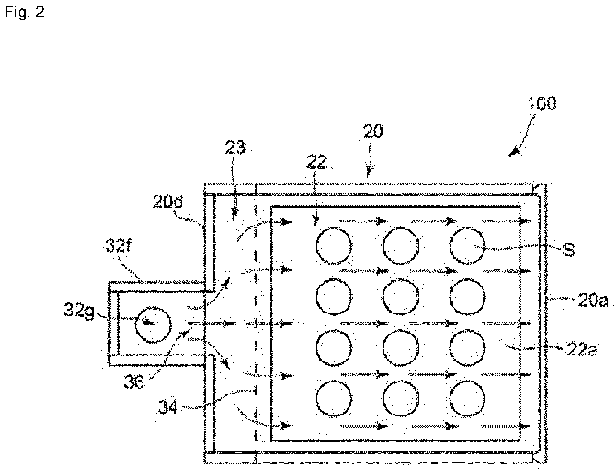

An incubator according to one embodiment of the present invention will be described with reference to the drawings. FIG. 1 is a cross-sectional view illustrating the inside of the incubator according to one embodiment of the present invention seen from the side. FIG. 2 is a cross-sectional view illustrating the inside of the incubator seen from above. In FIGS. 1 and 2, an incubator 100 is configured by a mount 10 placed on the floor, a culture vessel 20 mounted on the mount 10, a circulation device 30 for circulating the air inside the culture vessel 20, a temperature-regulating device 40 for regulating the temperature of the air inside the culture vessel 20, a gas supplying device 50 for supplying a decontamination gas or a humidifying water vapor to the inside of the culture vessel, and a decontamination gas decomposition device 80 for decomposing the residual decontamination gas in the air inside the culture vessel.

The culture vessel 20 has an outer wall and an inner wall, each covered with a metal plate made of stainless steel, between which an insulating material is filled to form an insulating wall. A heater for heating the inside of the culture vessel 20 may be provided inside the insulating wall. A front wall portion of the culture vessel 20 is provided with an openable/closable insulating door 20a, which can maintain an internal sterile environment (for example, Grade A) by achieving airtightness from the external environment when the door is closed. Additionally, the culture vessel 20 is provided with sensors for detecting the pressure, temperature, humidity, carbon dioxide gas concentration, and as needed the nitrogen gas concentration inside the vessel (each not shown).

A top wall portion 20b of the culture vessel 20 is provided with an air supply device 21 for regulating the air pressure inside the culture vessel 20 (an air exhaust device will be described later). The air supply device 21 is composed of an air-intake pipe 21a, an electromagnetic valve 21b provided in its conduit line, a disk filter 21c and an air-supply fan (not shown), and the air outside the culture vessel 20 is supplied to the inside of the culture vessel 20 as needed to keep a higher air pressure inside the culture vessel 20 than in the external environment.

The inside of the culture vessel 20 is separated into a front space 22 and a rear space 23 by a straightening plate (later-described) included in the circulation device 30. The front space 22 of the culture vessel 20 is a cultivation compartment, which is separated into 4 vertical spaces by 4 horizontally disposed shelf boards 22a (see FIG. 1). These shelf board 22a each have an upper surface, on which 12 petri dishes S each filled with a culture fluid for culturing cells are placed (see FIG. 2) . In addition, the 4 shelf boards 22a are configured, each as an integrated sliding shelf stand, to allow themselves to enter or leave the culture vessel 20 through the insulating door 20a.

The circulation device 30 includes a circulation fan 31 for circulating the air inside the culture vessel 20, a circulation passage 32 (32a to 32f) for circulating the air inside the culture vessel 20 by operating the circulation fan 31, a HEPA filter 33 provided in the circulation passage 32 (between 32e and 32f), and a straightening plate 34 for straightening the flow of the air supplied inside the culture vessel 20.

The circulation fan 31 is provided in communication with the circulation passage 32 (between 32a and 32b) to circulate the air inside the culture vessel 20 by operating the circulation fan 31. The type of the circulation fan 31 is not particularly restricted, and it may be any type so long as it provides a uniform force of wind.

The circulation passage 32 is provided at a lower portion of the culture vessel 20 (inside the mount 10) and at a back portion of the culture vessel 20 (see FIG. 1) . The air sucked into the circulation fan 31 flows from a suction port 35 that is opened at a bottom wall portion 20c (a portion near the insulating door 20a of the front space 22) of the culture vessel 20 into the circulation passage 32a and the air flowing from a connection portion 32g of a circulation passage 32e and a circulation passage 32f into the circulation passage 32f through circulation passages 32b to 32e is discharged from a discharge port 36 that is opened at a back wall portion 20d of the culture vessel 20 into the inside of the rear space 23 of the culture vessel 20 through the circulation passage 32f.

Branch passages 81a, 81b included in the decontamination gas decomposition device 80 are provided in communication with the passage (32a) of the circulation passage 32 through selector valves 83a, 83b. A catalyst device 82 for decomposing a decontamination gas is provided in the branch passages 81a, 81b. The mechanism and the type of catalyst of the catalyst device 82 are not particularly restricted, and the device may be selected according to the type of a decontamination gas used. In this embodiment, the decontamination gas used is a hydrogen peroxide gas.

The HEPA filter 33 is provided in communication with the circulation passage 32 (between 32e and 32f) to not only clean the air circulating in the circulation passage 32 but also equalize the air supplied to the inside of the culture vessel 20. In place of the HEPA filter 33, a ULPA filter or other filter may be employed. Accordingly, the air discharged into the rear space 23 of the culture vessel 20 through the circulation passage 32 is supplied to the front space 22 (cultivation compartment) of the culture vessel 20 through the straightening plate 34.

The straightening plate 34, as described above, separates the inside of the culture vessel 20 into the front space 22 (cultivation compartment) and the rear space 23 (see FIGS. 1 and 2) . In addition, the air discharged into the rear space 23 through the circulation passage 32 is supplied to the front space 22 (cultivation compartment) through the straightening plate 34. The structure of the straightening plate 34 is not particularly restricted, and it may be any type so long as it straightens the flow of the air supplied to the inside of the culture vessel 20. In this embodiment, the straightening plate 34 is composed of a rectangular frame body made of stainless metal, a porous sheet covering one surface of the frame body (a surface on the rear space 23 side), and a slit plate covering the other surface of the frame body (a surface on the cultivation compartment side) (each not shown).

The slit plate is provided with a plurality of thin grooves in parallel horizontally, the thin grooves passing through the slit plate between the front surface and the back surface thereof. The air supplied from the rear space 23 side to the front space 22 (cultivation compartment) side forms a unidirectional air flow (i.e., laminar flow) horizontally flowing through an internal space of the culture vessel 20 by a flow straightening action of the straightening plate 34. Specifically, the flow of the air discharged into the rear space 23 is straightened first through the porous sheet. The straightened air flow is further straightened through the slit plate to flow from each slit into the internal space of the culture vessel 20 as a unidirectional air flow. Inventors of the present invention describe the structure of the straightening plate in detail in a previous patent application (JP-A-2015-206854).

In this embodiment, the circulation passage 32b at a rear portion of the circulation fan 31 in the circulation passage 32 is provided with an air exhaust device 37. The air exhaust device 37 is composed of an exhaust pipe 37a, an electromagnetic valve 37b provided in its conduit line, a disk filter 37c and an exhaust fan (not shown) to exhaust the air inside the culture vessel 20 to the outside of the culture vessel 20 as needed. The air exhaust device 37 is operated and controlled by a microcomputer (not shown) in conjunction with the above pressure sensor (not shown) to keep a higher and more stable air pressure inside the culture vessel 20 than in the external environment in conjunction with the above air supply device 21.

The temperature-regulating device 40 is operated so as to stably maintain the temperature condition (culture condition) inside the culture vessel 20. The structure of the temperature-regulating device 40 is not particularly restricted, but in this embodiment, it is a temperature regulator composed of a Peltier element. In this embodiment, the temperature-regulating device 40 is provided in the circulation passage 32 (between 32d and 32e). The microcomputer (not shown) in conjunction with the above temperature sensor (not shown) can control the amount of the current supplied to the Peltier element and change the polarity of the current supplied to raise and lower the temperature, and thus precisely maintain the set temperature (in this embodiment, 37 °C). For a temperature-regulating device, an electric heater such as a rod-shaped sheath heater may be introduced inside a circulation passage or a culture vessel in place of a Peltier element.

The gas supplying device 50 is operated to supply a decontamination gas (in this embodiment, a hydrogen peroxide gas) to the inside of the culture vessel 20 when the inside of the culture vessel 20 is decontaminated before cells are cultured. In this embodiment, a proper amount of decontamination gas can be supplied to an incubator having a small volume without connecting the culture vessel 20 and the external decontamination device by including the gas supplying device 50. Accordingly, a sufficient decontamination effect can be provided, resulting in no excessive condensation of a decontamination liquid inside a culture vessel.

Also, the gas supplying device 50 is operated to supply a water vapor for stably maintaining the humidity condition (culture condition) inside the culture vessel 20 to the inside of the culture vessel 20 when cells are cultured after the autonomous decontamination. It is essential to stably maintain the temperature condition and the humidity condition in the culture conditions of the culture vessel 20, and in particular, the humidity condition must be kept at a high relative humidity of 95 to 100 %RH. Such a high humidity condition causes condensation due to slight changes in the temperature. To avoid this drawback, the microcomputer (not shown) is operated and controlled in conjunction with the above pressure sensor, the temperature sensor and the humidity sensor (each not shown) to stably maintain the temperature and humidity inside the culture vessel 20 in conjunction with the above air supply device 21, the air exhaust device 37, the temperature-regulating device 40 and the humidifier 50.

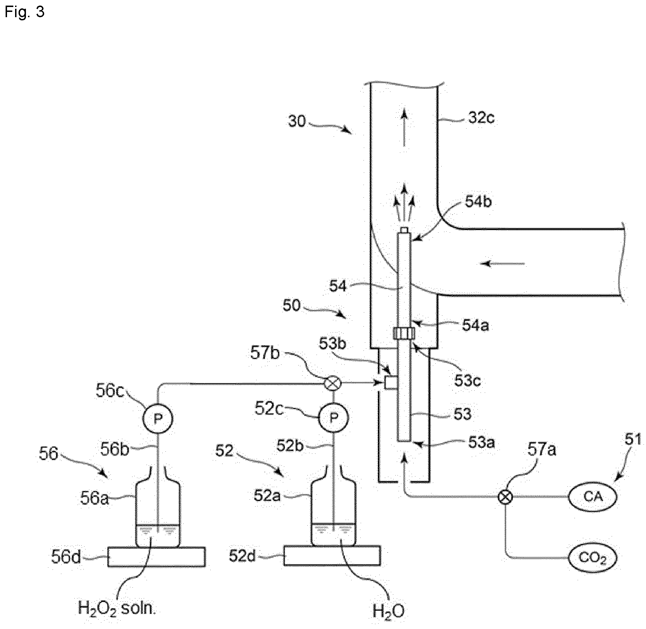

Accordingly, in this embodiment, a proper amount of decontamination gas is supplied to the inside of the culture vessel 20, and a specific gas supply mechanism capable of supplying a trace of water vapor is employed. The gas supplying device 50 is provided in the circulation passage 32 (at the position of 32c) to supply a decontamination gas or a humidifying water vapor to the air circulating in the circulation passage 32. FIG. 3 is a cross-sectional view illustrating a gas supplying device included in the incubator according to this embodiment. The gas supplying device 50 is composed of a compressed gas generator 51, a decontamination liquid supplying device 56, a water supplying device 52, a mixed gas/liquid regulator 53 and a vaporizer 54.

The compressed gas generator 51 used is a compressor for generating compressed air. In order to regulate the carbon dioxide gas concentration, the nitrogen gas concentration or other parameters in the culture vessel, these gases may be mixed with the compressed air. In FIG. 3, with compressed air CA as a main gas, a mixing valve 57a is configured to mix carbon dioxide gas CO₂ for supply as needed.

The decontamination liquid supplying device 56 is composed of a decontamination liquid tank 56a, a decontamination liquid pipe 56b, a decontamination liquid pump 56c and a load cell 56d. A decontamination liquid (in this embodiment, a hydrogen peroxide solution) is reserved inside the decontamination liquid tank 56a, which is placed on an upper surface of the load cell 56d. The decontamination liquid pipe 56b is provided such that one end portion thereof is inserted into a hydrogen peroxide solution inside the decontamination liquid tank 56a and the other end portion thereof is connected to the mixed gas/liquid regulator 53 through a selector valve 57b. Also, the decontamination liquid pump 56c is linked to a conduit line of the decontamination liquid pipe 56b.

Herein, the concentration of the hydrogen peroxide solution reserved in the decontamination liquid tank 56a is not particularly restricted, and in general, it is preferably 30 to 35 % by weight in view of hazardous materials in use.

In addition, the structure of the decontamination liquid pump 56c is not particularly restricted, but a proper amount of hydrogen peroxide solution can preferably be supplied according to the volume of the culture vessel 20. For example, a volumetric pump or other pump is preferably employed. In this embodiment, the decontamination liquid pump 56c used is a peristaltic pump as a type of volumetric pump. In this embodiment, the amount of hydrogen peroxide solution supplied by the decontamination liquid pump 56c is not particularly restricted, but for example, the amount of hydrogen peroxide gas supplied is preferably in the range of 0.1 g/min to 10 g/min. A proper amount of decontamination gas can be supplied to the culture vessel 20 having a small volume by appropriately controlling the amount of hydrogen peroxide solution. Accordingly, a sufficient decontamination effect can be provided, and no excessive condensation of a decontamination liquid occurs inside a culture vessel.

The water supplying device 52 is composed of a water storage tank 52a, a water supply pipe 52b, a feed pump 52c and a load cell 52d. Clean water is reserved inside the water storage tank 52a, which is placed on an upper surface of the load cell 52d. The water supply pipe 52b is provided such that one end portion thereof is inserted into the water inside the water storage tank 52a and the other end portion thereof is connected to the mixed gas/liquid regulator 53 through the selector valve 57b. Also, the feed pump 52c is linked to a conduit line of the water supply pipe 52b.

The structure of the feed pump 52c is not particularly restricted, but a trace of water can preferably be supplied in accordance with slight changes in the humidity sensor. For example, a volumetric pump or other pump is preferably employed. In this embodiment, the feed pump 52c used is a peristaltic pump as a type of volumetric pump. In this embodiment, the amount of water supplied by the feed pump 52c is not particularly restricted, but for example, is preferably in the range of 1 g/h to 60 g/h. Variations in the humidity inside the culture vessel 20 can be smoothed by controlling the amount of a trace of water supplied. The amount of water supplied can be detected by a volumetric pump and also the load cell 52d.

In this embodiment, the gas supplying device 50 can function both during an autonomous decontamination and when cells are cultured by including the decontamination liquid supplying device 56 and the water supplying device 52. The use of a selector valve 57b connects the decontamination liquid supplying device 56 and the mixed gas/liquid regulator 53 during an autonomous decontamination before cells are cultured, and connects the water supplying device 52 and the mixed gas/liquid regulator 53 when cells are cultured after the autonomous decontamination.

The mixed gas/liquid regulator 53 mixes the decontamination liquid supplied from the decontamination liquid supplying device 56 or the water supplied from the water supplying device 52 and the compressed air supplied from the compressed gas generator 51 to generate an atomized mixed gas/liquid (mist) . In this embodiment, the mixed gas/liquid regulator 53 used is an ejector. In FIG. 3, compressed air is supplied from the compressed gas generator 51 to a driving gas portion 53a of the mixed gas/liquid regulator 53. In addition, a hydrogen peroxide solution is supplied from the decontamination liquid supplying device 56 or water is supplied from the water supplying device 52 to a suction portion 53b of the mixed gas/liquid regulator 53. Meanwhile, the mixed gas/liquid (mist) , which is linked to the vaporizer 54 and atomized, is supplied to the vaporizer 54 from an ejection portion 53c of the mixed gas/liquid regulator 53.

The vaporizer 54 vaporizes the atomized mixed gas/liquid (mist) supplied from the mixed gas/liquid regulator 53 to generate a hydrogen peroxide gas or a water vapor. In FIG. 3, one end portion 54a of the vaporizer 54 is linked to the ejection portion 53c of the mixed gas/liquid regulator 53. The other end portion 54b of the vaporizer 54 is inserted into the inside of the circulation passage 32c to supply a decontamination hydrogen peroxide gas or a humidifying water vapor to the air circulating in the circulation passage 32 from the other end portion 54b.

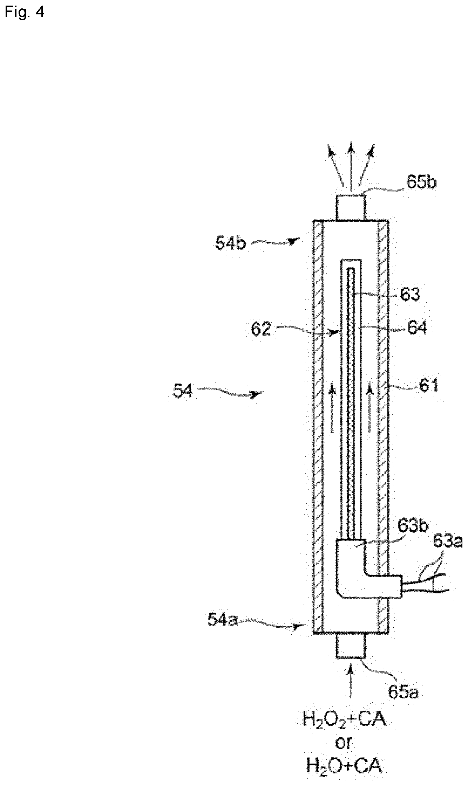

Herein, the structure of the vaporizer 54 used in this embodiment will be described. FIG. 4 is a cross-sectional view illustrating a vaporizer included in the gas supplying device according to one embodiment of the present invention. In FIG. 4, the vaporizer 54 is composed of a cylindrical outer cylinder pipe 61 extending from the one end portion 54a to the other end portion 54b and a heat generator 62 provided therein and in parallel to the longitudinal direction of the outer cylinder pipe 61. The outer cylinder pipe 61 is composed a stainless cylinder. The heat generator 62 includes a heater 63 extending in parallel to the longitudinal direction of the outer cylinder pipe 61. A gap is provided between an inner periphery of the outer cylinder pipe 61 and the heat generator 62, through which the mixed gas/liquid (mist) passes.

The heater 63 is disposed at one outer peripheral end portion of the outer cylinder pipe 61 (on the one end portion 54a side of the vaporizer 54) by a connecting terminal 63b made of silicon rubber to generate heat by power supply from a wire 63a. The heater 63, which is subjected to high temperature, has a surface coated with a quartz glass 64. In this embodiment, an inner periphery of the outer cylinder pipe 61 is also coated with quartz glass. The structure of the heater 63 is not particularly restricted, and it may be a stick-like heater or a coil-like heater. In addition, a surface of a heater or an inner periphery of an outer cylinder pipe should not always be coated with quartz glass, but in this embodiment, this is implemented to prevent dust generation and improve thermal efficiency.

In such a vaporizer 54, the atomized mixed gas/liquid (mist) supplied from the mixed gas/liquid regulator 53 is introduced from an inlet 65a that is opened at the one end portion 54a of the vaporizer 54 into the inside of the vaporizer 54. The mixed gas/liquid (mist) introduced into the inside of vaporizer 54 is heated by the heat generator 62 while it passes through the gap between the outer cylinder pipe 61 and the heat generator 62, and moves to a discharge port 65b that is opened at the other end portion 54b of the vaporizer 54. In the meantime, the hydrogen peroxide solution or water (liquid) in the mixed gas/liquid is heated by the heat generator 62 to be vaporized into a hydrogen peroxide gas or a water vapor (gas), and such a gas is discharged from the discharge port 65b. The temperature of the hydrogen peroxide gas or the water vapor of the discharge port 65b may be measured by a temperature sensor to control the temperature of the hydrogen peroxide gas or the water vapor discharged.

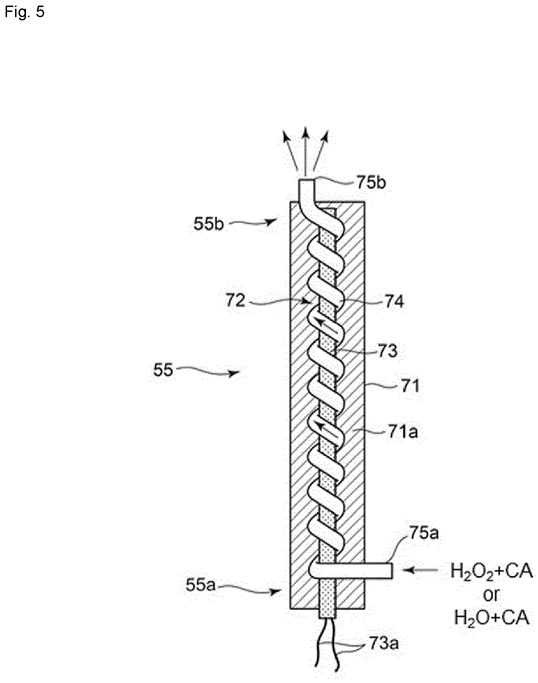

Herein, a vaporizer according to another embodiment of the present invention will be described. FIG. 5 is a cross-sectional view illustrating a vaporizer included in the humidifier according to another embodiment of the present invention. In FIG. 5, a vaporizer 55 is composed of a cylindrical outer cylinder pipe 71 extending from one end portion 55a to the other end portion 55b and a heat generator 72 provided therein and in parallel to the longitudinal direction of the outer cylinder pipe 71. The outer cylinder pipe 71 is composed of a stainless cylinder, with an insulating material 71a filled therein. The heat generator 72 includes a stick-like cartridge heater 73 extending in parallel to the longitudinal direction of the outer cylinder pipe 71 and an evaporation pipe 74 helically wound in the longitudinal direction along the periphery of the cartridge heater 73. The cartridge heater 73 and the evaporation pipe 74 are coated with the insulating material 71a. The cartridge heater 73 is disposed at one end portion of the outer cylinder pipe 71 (on the one end portion 55a side of the vaporizer 55) to generate heat from power supply from a wire 73a.

In such a vaporizer 55, the atomized mixed gas/liquid (mist) supplied from the mixed gas/liquid regulator 53 is introduced from an inlet 75a of the evaporation pipe 74 that is opened on an outer peripheral of the one end portion 55a of the vaporizer 55 into the inside of the evaporation pipe 74. The mixed gas/liquid (mist) introduced into the inside of the evaporation pipe 74 is heated by the cartridge heater 73 in contact with the evaporation pipe 74 while it passes through the inside of the evaporation pipe 74, and moves to a discharge port 75b of the evaporation pipe 74 that is opened at the other end portion 55b of the vaporizer 55. In the meantime, the hydrogen peroxide solution or water (liquid) in the mixed gas/liquid is heated by the cartridge heater 73 to be vaporized into a hydrogen peroxide gas or a water vapor (gas), and such a gas is discharged from the discharge port 75b. The temperature of the hydrogen peroxide gas or the water vapor of the discharge port 75b may be measured by a temperature sensor to control the temperature of the water vapor discharged.

The use of a vaporizer 54 or 55 with such a configuration can effectively supply even a proper amount of hydrogen peroxide solution or a trace of water, for example. In particular, the vaporizer 54 or 55 can effectively be used to provide a trace of water supplied in the range of 1 g/h to 60 g/h in terms of thermal efficiency when cells are cultured, and variations in the humidity inside the culture vessel 20 can be smoothed. In addition, the mixed gas/liquid is heated by the heat generator 62 or 72 to be converted into a high-temperature water vapor. In this embodiment, the temperature of the water vapor generated by the vaporizer 54 or 55 is controlled at 100 °C or higher in order to assuredly guarantee a sterilized state of the water vapor. The resulting sterilized water vapor generated by the vaporizer 54 or 55 can directly be supplied to the air circulating in the circulation passage 32 without passing through an air filter.

The high-temperature water vapor discharged by the vaporizer 54 or 55 is in an ultra-low quantity when cells are cultured, resulting in no large variations in the temperature of the air circulating in the circulation passage 32. Furthermore, in this embodiment, the Peltier element of the temperature regulator 40 provided in the circulation passage 32 constantly regulates the temperature (for both temperature rises and reduction) (see FIG. 1). Accordingly, the humidity and temperature environment inside the culture vessel 20 may be controlled, depending on the amount of water vapor discharged by the vaporizer 54 or 55 and the vapor temperature. Thus, a sterile environment of Grade A is maintained even in this embodiment where a water vapor is supplied from the external environment to the inside of the culture vessel 20 that is airtightly sealed from the external environment to maintain the internal sterile environment (for example, Grade A).

Subsequently, regarding the incubator 100 according to this embodiment as above configured, the air flow during the operation of an autonomous decontamination process and the operation of the incubator 100 will be described. First in FIG. 1, while the air supply device 21 and the air exhaust device 37 are not operated, the electromagnetic valve 21b and the electromagnetic valve 37b are closed and the inside of the culture vessel 20 and the circulation passage 32 is closed. The branch passages 81a, 81b are closed by the selector valves 83a, 83b provided in the passage (32a) of the circulation passage 32 not to allow the air circulating inside to be supplied to the decontamination gas decomposition device 80.

In this state, the air inside the culture vessel 20 and the circulation passage 32 circulates when the circulation fan 31 is operated. First, the air of the front space 22 (cultivation compartment of the culture vessel 20 flows from the suction port 35 that is opened at the bottom wall portion 20c of the culture vessel 20 (the portion near the insulating door 20a of the front space 22) into the circulation passage 32. Subsequently, the air flowing in the circulation passage 32 is discharged from the discharge port 36 that is opened at the back wall portion 20d of the culture vessel 20 from the circulation passage 32f into the inside of the rear space 23 of the culture vessel 20. In FIG. 1, the air flow flowing in the circulation passage 32 is indicated by arrows.

Herein, a hydrogen peroxide gas is supplied to the air flowing in the circulation passage 32. The gas supplying device 50 (herein, the decontamination liquid supplying device 56 is operated) is controlled by a preprogrammed microcomputer (not shown) to supply a proper amount of hydrogen peroxide gas with proper timing. The temperature is regulated by the temperature-regulating device 40 to control the decontamination temperature properly.

Subsequently, the air containing the hydrogen peroxide gas discharged into the inside of the rear space 23 of the culture vessel 20 is supplied to the front space 22 (cultivation compartment) of the culture vessel 20 after the straightening plate 34 straightens the gas flow. The air containing the hydrogen peroxide gas supplied to the front space 22 (cultivation compartment) through the straightening plate 34 forms a unidirectional air flow (laminar flow) traveling in the front space 22 (cultivation compartment) horizontally (left to right in FIGS. 1 and 2). In FIGS. 1 and 2, the air flow in the front space 22 (cultivation compartment) is indicated by arrows.

In FIG. 1, the air containing the hydrogen peroxide gas discharged through the straightening plate 34 flows into each of the top wall portion 20b, the bottom wall portion 20c and 4 chambers divided by 4 shelf boards 22a horizontally (left to right in FIG. 1). Accordingly, the air containing the hydrogen peroxide gas circulates inside the culture vessel 20 and the circulation passage 32 to decontaminate these spaces. Accordingly, the decontamination stage is completed in a predetermined period of time.

Subsequently, an operation for removing the residual hydrogen peroxide gas inside the culture vessel 20 and the circulation passage 32 will be performed. First, in FIG. 1, the selector valves 83a, 83b provided in the passage (32a) of the circulation passage 32 release the branch passages 81a, 81b to supply the air containing the hydrogen peroxide gas circulating inside to the decontamination gas decomposition device 80. In this state, the hydrogen peroxide gas contained in the air inside the culture vessel 20 and the circulation passage 32 is gradually decomposed as the circulation fan 31 is operated. Finally, the inside of the culture vessel 20 and the circulation passage 32 is aerated to complete the decontamination process. Thus, in this embodiment, the present invention can provide an incubator having an autonomous decontamination function that saves the need for connection to an external decontamination device.

Subsequently, regarding the incubator 100 according to this embodiment as above configured, the air flow during the operation of a cell culture process and the operation of the incubator 100 will be described. In FIG. 1, the inside of the culture vessel 20 and the circulation passage 32 is kept in a sterile and dust-free state by the above autonomous decontamination. Also, a higher air pressure inside the culture vessel 20 is maintained than in the external environment by operating the air supply device 21 and the air exhaust device 37. Accordingly, the inside of the culture vessel 20 is kept in the Grade A according to GMP standards.

In addition, the temperature inside the culture vessel 20 is maintained at 37±0.5 °C. The humidity inside the culture vessel 20 is maintained at 95 %RH or more to avoid variations in the osmotic pressure due to evaporation of a culture fluid. The carbon dioxide gas concentration inside the culture vessel 20 (nitrogen gas concentration as needed) is maintained at a required concentration to ensure the optimum condition for cell culture.

In this state, the air of the front space 22 (cultivation compartment) of the culture vessel 20 flows from the suction port 35 that is opened at the bottom wall portion 20c of the culture vessel 20 (the portion near the insulating door 20a of the front space 22) into the circulation passage 32 when the circulation fan 31 is operated. The gas supplying device 50 (herein, the water supplying device 52 is operated) , the temperature-regulating device 40 and the HEPA filter 33 are controlled by a microcomputer (not shown) in conjunction with the sensors to regulate the humidity, to regulate the temperature and to clean and equalize the air flowing in the circulation passage 32, respectively. Subsequently, the air flowing in the circulation passage 32 is discharged from the discharge port 36 that is opened at the back wall portion 20d of the culture vessel 20 from the circulation passage 32f to the inside of the rear space 23 of the culture vessel 20. In FIG. 1, the air flow flowing in the circulation passage 32 is indicated by arrows.

Herein, as described above, the inside of the culture vessel 20 is maintained in a sterile and dust-free state. However, the inside of the culture vessel 20 can be kept in a sterile and dust-free more completely by further allowing the HEPA filter 33 of the circulation passage 32 to clean the air. For example, the inside of the culture vessel 20 is kept in a sterile and dust-free state by the action of the HEPA filter 33 even in cases where foreign objects such as microorganisms are present inside the circulation passage 32 due to some hazards.

The air discharged into the inside of the rear space 23 of the culture vessel 20 is supplied to the front space 22 (cultivation compartment) of the culture vessel 20 after the straightening plate 34 straightens the air flow. The air supplied to the front space 22 (cultivation compartment) through the straightening plate 34 forms a unidirectional air flow (laminar flow) traveling in the front space 22 (cultivation compartment) horizontally (left to right in FIGS. 1 and 2) . The temperature, humidity and carbon dioxide gas concentration of the unidirectional air flow flowing in the front space 22 (cultivation compartment) are correctly maintained as the set conditions. In FIGS. 1 and 2, the air flow in the front space 22 (cultivation compartment) is indicated by arrows.

In FIG. 1, the air discharged through the straightening plate 34 flows into each of the top wall portion 20b, the bottom wall portion 20c and 4 chambers divided by 4 shelf boards 22a horizontally (left to right in FIG. 1). The temperature, humidity and flow velocity of the air are maintained at certain levels. In each of the 4 chambers divided, a petri dish S filled with a culture fluid is placed on a shelf board (in this embodiment, 12 dishes placed on each shelf board) . Therefore, a unidirectional air flow with fixed temperature, humidity and carbon dioxide gas concentration is allowed to flow at a constant flow velocity on the surface of each petri dish S (left to right in FIG. 2). Moreover, the temperature, humidity and carbon dioxide gas concentration of the unidirectional air flow are detected by the above temperature sensor, humidity sensor, and carbon dioxide gas concentration sensor inside the culture vessel 20.

Herein, the petri dishes S are made of glass, whose heat transfer coefficient (K value) is not small. However, conventional approaches of heating by radiation or agitation in incubators require an extremely long period of time for raising the temperature of a culture fluid inside the petri dish S to meet the culture condition. On the other hand, in this embodiment, a unidirectional air flow with a constant temperature is constantly supplied, thereby increasing heat supply for the petri dish S. Therefore, in this embodiment, as the apparent heat transfer coefficient (K value) of the petri dish S further increases, the temperature of the culture fluid inside the petri dish S can be raised in a short period of time to meet the culture condition. Thus, in this embodiment, the present invention can provide an incubator capable of raising the temperature inside the petri dish containing a subject to be cultured to a predetermined temperature in a short period of time.

As described above, the present invention can provide an incubator having not only a favorable cell culture function but also an autonomous decontamination function that saves the need for connection to an external decontamination device.

The present invention is achieved not only by the above embodiment, but also by the following various alternatives.
(1) In the above embodiment, the positions of an air supply device, an air exhaust device, a temperature-regulating device and a gas supplying device are specified, but the configuration is not restricted thereto, and a culture vessel or a circulation passage may be provided at other locations.
(2) In the above embodiment, a temperature sensor, a humidity sensor and a carbon dioxide gas concentration sensor are located in the culture vessel, but the configuration is not restricted thereto, and they may be provided in the circulation passage.
(3) In the above embodiment, the temperature-regulating device of the air used is a Peltier element, but the configuration is not restricted thereto, and an electric heater such as a rod-shaped sheath heater may be employed inside the circulation passage or the culture vessel.
(4) In the above embodiment, the culture vessel has 4 shelf stages, but the configuration is not restricted thereto, and it may be provided with 3 or less or 5 or more shelf stages .
(5) In the above embodiment, a carbon dioxide gas supplying means is provided to regulate the carbon dioxide gas concentration inside the culture vessel, but the configuration is not restricted thereto, and no carbon dioxide gas supplying means may be provided, depending on a culture condition.
(6) In the above embodiment, the carbon dioxide gas supplying means is provided to regulate the carbon dioxide gas concentration inside the culture vessel, but the configuration is not restricted thereto, and a nitrogen gas supplying means may be provided to regulate the nitrogen gas concentration inside the culture vessel, in addition to or in place of the carbon dioxide gas supplying means, depending on a culture condition.
(7) In the above embodiment, a straightening plate composed of one porous sheet and one slit sheet is employed, but the configuration is not restricted thereto, and one or more porous sheets may be employed. In this case, the porous sheet used is preferably a screen fabric, a porous ceramic sheet or the like.
(8) In the above embodiment, a straightening plate composed of one porous sheet and one slit sheet is employed, but the configuration is not restricted thereto, and an incubator may be configured without using a straightening plate.
(9) In the above embodiment, any door for allowing a culture container to enter or leave the culture vessel is not specifically described, but a Rapido Transfer Port (RTP) -like door that can be connected to an isolator or the like in a sterile manner may be provided. Such a door is not restricted to a RTP door, and an incubator may be configured to include a door having a mechanism capable of connection in a sterile manner or a simple openable/closable door.

### REFERENCE SIGNS LIST

100...Incubator, 10...Mount, 20...Culture vessel, 20a...Insulating door,
20b to 20d...Insulating wall, 21...Air supply device,
21a ... Air-intake pipe, 21b...Electromagnetic valve,
21c...Disk filter, 22...Front space, 22a...Shelf board, 23...Rear space,
30...Circulation device, 31...Circulation fan, 32 (32a to 32f) ...Circulation passage,
33...HEPA filter, 34...Straightening plate, 35...Suction port,
36...Discharge port,
37...Air exhaust device, 37a...Air-intake pipe,
37b...Electromagnetic valve, 37c...Disk filter,
40...Temperature regulator, 50...Gas supplying device, 51...Compressed gas generator, 52...Water supplying device,
52a...Water storage tank, 52b...Water supply pipe, 52c...Feed pump,
52d, 56d...Load cell, 56...Decontamination liquid supplying device, 56a...Decontamination liquid tank,
56b...Decontamination liquid pipe, 56c...Decontamination liquid pump, 53...Mixed gas/liquid regulator,
54, 55...Vaporizer, 61, 71...Outer cylinder pipe, 62, 72...Heat generator,
63, 73...Heater, 64...Quartz glass, 74...Evaporation pipe,
80...Decontamination gas decomposition device, 81 (81a, 81b)...Branch passage,
82...Catalyst device, 83a, 83b...Selector valve, S...Petri dish.

## Claims

1. An incubator having not only a cell culture function but also an autonomous decontamination function, the incubator comprising:
a culture vessel having an insulating door and an insulating wall; a circulating means having a circulation passage for circulating the air inside the culture vessel; a temperature-regulating means for regulating the temperature of the air inside the culture vessel; and a gas supplying means for supplying a decontamination gas or a humidifying water vapor to the air inside the culture vessel, wherein
the gas supplying means includes: a compressed gas generating means for generating a compressed gas; a decontamination solution supplying means for supplying a decontamination liquid; a water supplying means for supplying water; a mixed gas/liquid regulator for regulating a mixed gas/liquid composed of the compressed gas and the decontamination liquid or the water in mixture; and a vaporizer for vaporizing the mixed gas/liquid to generate a decontamination gas or a water vapor, wherein
the gas supplying means acts as a decontamination means for supplying the decontamination gas to the air circulating in the circulation passage during an autonomous decontamination before cells are cultured, and
the gas supplying means acts as a humidifying means for humidifying the air circulating in the circulation passage when cells are cultured after the autonomous decontamination.

2. The incubator according to claim 1, comprising a decontamination gas decomposing means for decomposing a decontamination gas, wherein
the decontamination gas decomposing means includes a branch passage branching off from the circulation passage and a catalyst means for decomposing a decontamination gas in the branch passage, wherein
the branch passage is closed to prevent the air circulating in the circulation passage from flowing into the branch passage during an autonomous decontamination before cells are cultured, and
the branch passage is opened to allow the air circulating in the circulation passage to flow into the branch passage and allow the catalyst means to decompose the residual decontamination gas in the air when the autonomous decontamination is completed.

3. The incubator according to claim 1 or 2, wherein
the compressed gas is a single gas or a mixed gas composed of one or more of air, carbon dioxide and nitrogen.

4. The incubator according to any one of claims 1 to 3, wherein
the amount of a decontamination gas supplied by the vaporizer is in the range of 0.1 g/min to 10 g/min, and the amount of a water vapor supplied by the vaporizer is in the range of 1 g/h to 60 g/h.

5. The incubator according to any one of claims 1 to 4, wherein
the vaporizer includes a cylindrical outer cylinder pipe and a heat generator provided inside the outer cylinder pipe and parallel to the longitudinal direction of the outer cylinder pipe, wherein
the mixed gas/liquid is heated while it passes between the outer cylinder pipe and the heat generator to supply a decontamination gas or a sterilized water vapor.

6. The incubator according to claim 5, wherein
the heat generator is a heater coated with a quartz glass.

7. The incubator according to any one of claims 1 to 4, wherein
the vaporizer includes a cylindrical outer cylinder pipe and a heat generator provided inside the outer cylinder pipe and parallel to the longitudinal direction of the outer cylinder pipe, wherein
the heat generator includes a stick-like heater arranged in the longitudinal direction thereof and an evaporation pipe helically wound along the periphery of the heater in the longitudinal direction, and
the mixed gas/liquid is heated while it passes inside the evaporation pipe to supply a decontamination gas or a sterilized water vapor.
